# EUROPEAN PATENT SPECIFICATION

(11) **EP 0 621 038 B1**
(45) Date of publication and mention of the grant of the patent: **02.12.1998**
(21) Application number: 94104300.2
(22) Date of filing: 18.03.1994
(51) Int. Cl.: A61K 33/06, A61K 33/12, A61K 33/00

(54) **Pharmaceutical compositions containing silicate polymers**
Silikat-Polymer enthaltende pharmazeutische Zusammensetzungen
Compositions pharmaceutiques renfermant des polymères du silicate

(30) Priority: 19.03.1993 JP 85322/93
(43) Date of publication of application: 26.10.1994
(73) Proprietor: NIPPON ZOKI PHARMACEUTICAL CO., LTD., Chuo-ku, Osaka (JP)
(72) Inventor: Konishi, Jin-emon, Musashino-shi, Tokyo (JP); Hamada, Giichi, Nishinomiya-shi, Hyogo-ken (JP)
(74) Representative: Hansen, Bernd, Dr. Dipl.-Chem.

(56) References cited:
- CHEM.PHARM.BULL. vol. 27, no. 8 , 1979 pages 1733 - 9
- Derwent Publications Ltd., London, GB; AN 82-10241J & JP-A-57 183 720 (MITANI J.) 12 November 1982
- PATENT ABSTRACTS OF JAPAN vol. 7, no. 255 (C-189)6 October 1983 & JP-A-58 121 217 (KAGITANI TAKEO) 19 July 1983
- PATENT ABSTRACTS OF JAPAN vol. 11, no. 371 (C-462)3 December 1987 & JP-A-62 145 022 (SOFUTO SHIRIKA) 29 June 1987
- PATENT ABSTRACTS OF JAPAN vol. 15, no. 474 (C-890)3 December 1991 & JP-A-32 004 803 (SHISEIDO CO LTD) 6 September 1991

## Description

The present invention relates to silicate polymers which regulate the physiological function of a living body, a method for manufacturing thereof, and a pharmaceutical composition containing said polymer as an active ingredient.

A living body maintains its life as an independent organism by controlling physical and chemical conditions of the body within a certain physiological state, and responding to alterations of the internal and external environment.

Such a mechanism maintaining the homeostasis of a living body is effected by the living cells of the body, particularly through their cell membranes. The cell membrane, as is well known, is made up of a phospholipid bilayer, and recognizes various molecules with its surface protein, which acts as a receptor, and selectively transmits chemicals and ions to achieve intra- and extra-cellular equilibria of substances and chemical conditions. These mechanisms maintain the physiological balance of the cell and cause it to act normally.

If said balance is disturbed for any reason, however, various troubles may arise; for instance, a change in the amount of fatty acid components of phospholipids, i.e. a decrease of unsaturated fatty acids or an increase in the amount of unsaturated and saturated fatty acids, may make the cell membrane solid and lower the fluidity of the membrane.

The alteration of the membrane fluidity may result in the hypofunction of the membrane receptors as well as of channels for sodium, potassium, calcium and other ions, which eventually brings about functional disturbance of the cell.

Chem. Pharm. Bull, vol. 27, no. 8, (1979), pages 1733-9 discusses the effect of the polymerization degree of silicic acid on the gastrointestinal absorption of silicate in rats. Reference is made to the use of low molecular weight polysilicates as antacid agents in order to avoid calculi.

JP-A-57 183720 discloses that a drug formed from low water-solubility water glass powder increases the appetite of fish and can treat a fish disease such as white mould.

JP-A-58 121217 discloses that a liquid formed from *inter alia* water glass is effective for treating burns. The burned part is coated with a volatile solvent containing water glass with a brush, and the solution allowed to dry to form a protecting coating over the burned part.

The present inventors have conducted continued studies on the substances which enhance the naturally acquired healing power of a living body and contribute to normalization of functions of the body, focusing on the homeostatic mechanisms regulating and restoring neurological, endocrinological and immunological disturbances due to functional abnormality of cells in a morbid state, and, as a result thereof, have accomplished the present invention.

The present invention relates to water-soluble silicates which are activated by polymerization. It also relates to biologically active substances comprising said silicate polymers which regulate and maintain the function of a living body by restoring and normalizing the lowered cell functions due to diseases. It further relates to a pharmaceutical composition containing said silicate polymer as an active ingredient, and to a method for manufacturing the same.

Silicon is a natural element widely spread in organisms of the animal and plant kingdoms. In particular, it exists as silicate in animal tissues like hair, feather, bone and skin and is known as an essential element in osteogenesis. In animal tissues, it is involved in cross linkage of collagen tissues and is present in acidic mucopolysaccharide as one of the components. Silicon is thus an essential element for the living body. It is known, additionally, that silicon has pharmacological activities such as immuno-suppression through its anti-macrophage activity and anti-diabetic activity, when administered to animals.

Some silicates like magnesium silicate and aluminum silicate are widely used as anti-acid pharmaceutical products. However, it has not been reported that a silicate polymer has any specific pharmacological activities.

The objects of the present invention are to provide novel silicate polymes which regulate the function of a living body, a method for manufacturing the same and a pharmaceutical composition containing said polymer as an active ingredient.

According to one aspect, the present invention provides a pharmaceutical composition containing a water-soluble silicate polymer having a molecular weight of 4,800-2,000,000 and a degree of polymerization of 75-33,000 as an active ingredient and a pharmaceutically acceptable carrier.

The water-soluble silicate polymer provided by this invention regulates the function of a living body by restoring and normalizing lowered cell functions caused by diseases. Accordingly, a further aspect of the invention provides the use of a water-soluble silicate polymer having a molecular weight of 4,800-2,000,000 and a degree of polymerization of 75-33,000 for the manufacture of a medicament effective in regulating the function of a living body. The medicament can act as an anti-allergy agent, an anti-inflammatory agent, an agent for improving peripheral blood circulation, an agent for improving paresthesia or an analgesic agent.

The silicate polymers provided by this invention are polymerized substances of silicic acids or silicates. The silicic acids include orthosilicic acid, metasilicic acid, mesodisilicic acid, mesotrisilicic acid, etc. The silicates are the salts of said silicic acids, for example, salts with alkali metals such as sodium and potassium. A material containing silicic acid, for instance, water glass which is a concentrated aqueous solution of alkali salts of silicic acid, may also be used. Further, a silicate solution prepared by heating and dissolving silicon oxide in an alkaline aqueous solution may be utilized.

The silicate polymers provided by this invention have a molecular weight of 4,800 to 2,000,000, preferably, 20,000 to 1,000,000. The molecular weight can be determined by gel-filtration, ultrafiltration, electrophoresis and the like.

The silicate polymers provided by this invention have a degree of polymerization of 75 to 33,000, preferably, 490 to 16,500.

According to a further aspect, the present invention provides a process for manufacturing a water-soluble silicate polymer having a molecular weight of 4,800-2,000,000 and a degree of polymerization of 75-33,000, comprising the steps of dissolving a silicate in an aqueous solvent, adding a saccharide to the silicate solution, adjusting the pH of the solution to 4-10, and then drying the solution.

The silicate dissolved in the aqueous solution may be sodium orthosilicate, sodium metasilicate, potassium orthosilicate or potassium metasilicate, or silicic acid containing material such as water glass. Since said aqueous solution of silicate displays a high pH value, the pH of the solution is adjusted to 4-10, preferably 4-9.5, by use of a conventional acid such a hydrochloric acid, sulfuric acid or acetic acid. A saccharide such as lactose, mannitol, sorbitol, sucrose, glucose, fructose or galactose is added to the aqueous solution. A salt such as sodium chloride, potassium chloride or sodium sulfate may also be added.

For the purpose of manufacturing a pharmaceutical composition of the invention, said aqueous solution of silicate is dried to a powder. The powderization may be carried out according to a conventional method such as heating or lyophilization. To give a preferred powder, for example, the solution is dried by heating at from 150 to 250°C. Also conventional lyophilization under reduced pressure may be used to powderize the solution.

The production of silicate polymers can be detected, for example, by measuring the molybdenum blue coloration. Namely, a solution of ammonium molybdate is added to the aqueous solution of silicate and then sulfite solution is added to produce a blue coloration. The blue coloration reduces as the polymerization of the silicate proceeds. Also, the silicate polymers so produced can be separated by gel-filtration under acidic conditions at high molecular bands. An aliquot of this separated fraction is decomposed under alkaline conditions to detect silicate by the molybdenum blue reaction.

### Examples

### Example 1

7.6 g of water glass (silicon; 1.2 g) was dissolved in 100 ml of water. 97.5 g of lactose was dissolved in 300 ml of water with heating. The water glass solution was mixed with the lactose solution, and then the pH was adjusted to 8.0 with diluted hydrochloric acid. The reaction mixture was dried at 200°C to give 90 g of a powder. The resulting silicate polymers of the present invention have the following physical properties and contain 12 mg of silicon per 1 g.
- Molecular weight:: 13,000 to 1,000,000
- Degree of Polymerization:: 210 to 16,500

### Example 2

12.9 g of sodium metasilicate (silicon; 1.2 g) was dissolved in 100 ml of water. 95.8 g of lactose was dissolved in 300 ml of water with heating. The aqueous solution of sodium metasilicate was mixed with the lactose solution, and then the pH was adjusted to 8.0 with diluted hydrochloric acid. The reaction mixture was dried at 200°C to give 90 g of a powder. The resulting silicate polymers of the present invention have the following physical properties and contain 12 mg of silicon per 1 g.
- Molecular weight:: 15,000 to 900,000
- Degree of Polymerization:: 250 to 15,000

### Example 3

5.9 g of sodium orthosilicate (silicon; 0.6 g) was dissolved in 100 ml of water. 99 g of mannitol was dissolved in 300 ml of water with heating. The aqueous solution of sodium orthosilicate was mixed with the mannitol solution, and then the pH was adjusted to 8.0 with diluted hydrochloric acid. The reaction mixture was dried at 200°C to give 88 g of a powder. The resulting silicate polymers of the present invention have the following physical properties and contain 6 mg of silicon per 1 g.
- Molecular weight:: 20,000 to 1,000,000
- Degree of Polymerization:: 330 to 16,500

### Example 4

1,89 g of water glass (silicon; 0.3 g) was dissolved in 100 ml of water. 500 ml of 20 % aqueous solution of lactose was added thereto, and then the pH was adjusted to 8.0 with diluted hydrochloric acid. Each 1.2 ml of the solution was pipetted into a vial and lyophilized. The resulting dry-powdered silicate polymers of this invention have the following physical properties and contain 0.6 mg of silicon per vial.
- Molecular weight:: 30,000 to 1,500,000
- Degree of Polymerization:: 490 to 25,000

In the following experiments, the amount of substance of the present invention was indicated as the amount of silicon (Si) contained therein, such as Si g/ml or Si mg/kg.

### 1. Inhibitory effect on mast cell degranulation:

An inhibitory effect of a substance of the invention on mast cell degranulation was determined according to a usual method. A solution of the substances was added to a cell suspension of rat peritoneal mast cells and incubated at 37°C for 10 minutes. To this cell suspension, a solution of Compound 48/80, a histamine releaser, was added. The reaction mixture was incubated for 10 minutes, and then the reaction was stopped by adding a cold buffer solution. The supernatant was separated by centrifuging, and the amount of histamine in it was measured by fluorometric methods. The inhibition rate of the substance on histamine release was determined according to a conventional method. The substance significantly inhibited the histamine release from mast cells.

An example of the results is shown in Table 1.

**Table 1**

| **Concentration of the substance of Example 1 (Si g/ml)** | **Inhibition rate on histamine release (%)** |
|---|---|
| 1 x 10⁻⁸ | 6.2 ± 2.0 |
| 1 x 10⁻⁷ | 7.6 ± 8.5 |
| 1 x 10⁻⁶ | 37.6 ± 0.7 |
| 1 x 10⁻⁵ | 83.0 ± 11.9 |
| 1 x 10⁻⁴ | 96.2 ± 0.9 |

### 2. Inhibitory effect on histamine release by antigen-antibody reaction:

Rats were passively sensitized by intraperitoneal injection of anti-ovalbumin serum (IgE-like antibody). Twenty-four hours thereafter, a suspension of peritoneal mast cells of the rats was prepared. To this cell suspension, a solution of the substance of the invention was added, and incubated for 10 minutes at 37°C. A solution of ovalbumin was then added to the mixture to induce antigen-antibody reaction. The reaction was stopped after 10 minutes and the inhibition rate of histamine release by the substance was measured, according to the methods described above, in 1. The substance significantly inhibited the histamine release from mast cells caused by antigen-antibody reaction.

An example of the results is shown in Table 2.

**Table 2**

| **Concentration of the substance of Example 2 (Si g/ml)** | **Inhibition rate on histamine release (%)** |
|---|---|
| 1 x 10⁻⁸ | 3.1 ± 1.1 |
| 1 x 10⁻⁷ | 16.6 ± 12.2 |
| 1 x 10⁻⁶ | 85.1 ± 5.9 |
| 1 x 10⁻⁵ | 96.5 ± 4.5 |
| 1 x 10⁻⁴ | 98.4 ± 2.8 |

### 3. Inhibitory effect on hyaluronidase activity:

A substance of the present invention was added to a solution containing hyaluronidase, and incubated at 37°C for 10 minutes. A solution of hyaluronic acid as a substrate was added to the solution to react with the enzyme for 15 minutes, and then the reaction was terminated by addition of a succinic acid-buffer solution. The absorbance of the reaction solution at 540 nm was measured. The inhibition rate of the substance to hyaluronidase was calculated in comparison with the absorbance of a control solution wherein a physiological saline solution was used instead of the tested substance. The substance showed inhibition of hyarulonidase activity dose-dependently.

An example of the results is shown in Table 3.

**Table 3**

| **Concentration of the substance of Example 2 (Si µg/ml)** | **Inhibition rate to hyaluronidase (%)** |
|---|---|
| 50 | 10.9 |
| 100 | 24.5 |
| 250 | 51.7 |
| 500 | 89.1 |

### 4. Inhibitory effect on vascular permeability:

Guinea pig nasal cavity was perfused with histamine solution, and the increase of nasal membrane permeability and of nasal secretion was investigated by measuring the amount of dye extravasated at the site according to the method of Kojima and Tsutsumi [Allergy, vol. 35, 180-187 (1986)]. A polyethylene tube was inserted into the nasal cavity from an incision in the trachea for perfusion, and a solution of dye was intravenously injected. A physiological saline solution was perfused at 1 ml per minute. The initial perfusate for 10 minutes was drained off from the nostrils for washing, and the perfusate of the following 20 minutes was collected (Perfusate A). A physiological saline solution containing histamine was then perfused for 10 minutes to collect the perfusate (Perfusate B). A normal saline solution was again perfused for 10 minutes to collect the perfusate (Perfusate C). Each perfusate was centrifuged and the absorbance of the supernatant was measured at 620 nm.

A substance of the invention, which was intraperitoneally administered 30 minutes before histamine perfusion, significantly suppressed the permeability of the nasal membrane to the dye.

An example of the results is shown in Table 4. The amount of dye in the perfusate was expressed as the absorbance.

**Table 4**

| Amount of dye in the perfusate | | | |
|---|---|---|---|
| | | Dose of the substance of Example 2 (Si mg/kg) | |
| Perfusate | Saline | 12.5 | 25.0 |
| A | 0.88 ± 0.44 | 0.45 ± 0.13 | 0.33 ± 0.09 |
| B | 4.07 ± 2.40 | 1.34 ± 1.12 | 0.75 ± 0.46 |
| C | 3.82 ± 2.05 | 1.72 ± 1.75 | 0.88 ± 0.56 |

### 5. Effect on disturbance of peripheral blood circulation:

The effect of a substance of the invention on the disturbance of peripheral blood circulation was determined, as an index of improving paresthesia. Quinoform was administered to rats intraperitoneally for 27 days in progressively increased amounts to cause disturbance of peripheral circulation. The hind limbs of the quinoform-treated rats were then exposed to cold water at 5°C for 2 minutes. The effect of the substance in improving peripheral blood circulation was evaluated by monitoring the recovering process of the limb temperature with thermographical image analysis. The substance was intravenously administered to rats consecutively for 7 days from the 21st day of quinoform treatment.

An example of the results is shown in Table 5. The dose of the substance is expressed as Si mg/kg and is shown in parenthesis.

**Table 5**

| | Average skin temperature (°C) of the hind limb at 15 minutes after exposure to cold water |
|---|---|
| Untreated group | 27.7 ± 0.7 |
| Control: quinoform-treated | 23.9 ± 0.5 |

| The substance of Example 1 | |
|---|---|
| (5) | 24.2 ± 0.2 |
| (10) | 26.3 ± 0.2 |
| (20) | 27.0 ± 0.3 |

### 6. Analgesic effect:

Analgesic effects of a substance of the present invention was studied by Randall-Selitto method using SART-Stressed mice prepared according to Kita, et al. [Folia Pharmacol. Jap., vol. 71, 195-210 (1975)]. The SART-stressed animal is an animal model of hyperalgesia, with lowered threshold to pain sensation, and highly responsive when applying pressure stimulus to the tail root of the animal by a Randall-Selitto device.

The effect of the substance was evaulated by an analgesic index: the quotient of the pain threshold after treatment divided by that before treatment. The lowered pain threshold of SART-stressed mice was elevated dose-dependently up to normal level, when the substance of the present invention was intraperitoneally administered.

An example of the results is shown in Table 6.

**Table 6**

| Dose of the substance of Example 3 (Si mg/kg) | Average pain threshold |
|---|---|
| 2.5 | 1.35 |
| 5.0 | 1.51 |
| 10.0 | 1.70 |

### 7. Acute toxicity:

Acute toxicity of a substance of the present invention was measured by administering the substance intravenously or intraperitoneally to a group of 10 ddY male mice. The LD₅₀ values of the substance were 60.4 Si mg/kg (i.v.) and 71.8 Si mg/kg (i.p.), respectively.

As apparently shown by the above-mentioned results of pharmacological studies, the silicate polymers of the present invention have biological activities which restore and normalize the abnormal functions of a living body due to diseases. For example, the substances of this invention show a therapeutic effect on diseased conditions such as allergy or inflammation, and elevate the lowered pain threshold in animals under stress.

The substances of the present invention have, as demonstrated, an effect on neurological, endocrinological and immunological systems and show an excellent activity in restoring and normalizing the impaired functions of living body due to diseases. They are very useful as drugs such as anti-allergic agents, anti-inflammatory agents, improving agents for peripheral blood circulation and paresthesia, and analgesic agents. Consequently, a pharmaceutical composition containing a substance of the invention as an active ingredient is useful for treating or preventing various diseases accompanying allergy, inflammation or pain, including bronchial asthma, pollinosis, allergic rhinitis, ulticaria, eczema, contact dermatitis, allergic conjunctivitis, gastritis, peptic ulcer, enterocolitis, idiopathic ulcerative colitis, rheumatoid arthritis, herpes, systemic lupus erythematosus, gastrointestinal neurosis, autonomic dystonia, vertigo, neuralgia, frozen shoulder, low back pain, headache, etc.

The substances of the present invention can be made into pharmaceuticals by combining with suitable carriers or diluents for medical use and can be made into pharmaceutical preparations by any conventional methods giving solid, semisolid, liquid or gaseous forms for oral or parenteral administration.

In manufacturing such preparations, the substances of the present invention may be used either solely or jointly in the form of a suitable combination. Alternatively, the substances may be combined with other pharmaceutically active components.

In the case of preparations for oral use, the substances of the present invention may be made into tablets, diluted powders, granules or capsules with or without suitable additives such as conventional fillers (e.g. lactose, mannitol, corn starch and potato starch) as well as binders (e.g. crystalline cellulose, cellulose derivatives, gum arabic, corn starch and gelatin), lubricants (e.g. talc and magnesium stearate), extenders, moisturizers, preservatives and perfumes.

Alternatively, the substances of the present invention may be mixed with a base material of fat/oil type (e.g. cacao butter), emulsifying base material, water-soluble base material (e.g. Macrogol), hydrophilic base material, etc. to give a suppository.

In the case of injections, the substances may be made into a solution or a suspension in aqueous solvents or nonaqueous ones such as distilled water for injection, physiological saline liquid, Ringer solution, plant oil, synthetic fatty acid glycerides, higher fatty acid esters and propylene glycol.

Further, depending upon the state of the patient, or the type of the disease, the substances may be made into other preparation forms such as ointments and eye drops which are most suitable for the therapy.

A desired dose of the substances of the present invention may vary depending upon the patient to be treated, preparation form, method of administration, period for administration, etc. In general, 1 µg/kg to 10 mg/kg per day may be given to an adult for achieving the desired effect.

In the case of parenteral administration such as injections, the desired dose may be 1/3 to 1/10 as large as an oral dose in general because of the difference of absorption.

Some prescriptions of the pharmaceutical compositions which contain the substances of the present invention as active ingredients are shown below as examples.

| Prescription example 1 (tablet) | |
|---|---|
| Component | Content in a tablet (mg) |
| substance of this invention | 100 |
| lactose | 58 |
| Crystalline cellulose | 40 |
| magnesium stearate | 2 |
| Total | 200 |

| Prescription example 2 (capsule) | |
|---|---|
| Component | Content in a capsule (mg) |
| substance of this invention | 50 |
| lactose | 78 |
| potato starch | 15 |
| magnesium stearate | 2 |
| talc | 5 |
| Total | 150 |

| Prescription example 3 (injection) | |
|---|---|
| Component | Content in an ampule (mg) |
| substance of this invention | 10 |
| saline | proper amount |
| Total | 1 ml |

| Prescription example 4 (ointment) | |
|---|---|
| Component | (g) |
| substance of this invention | 1 |
| glycerol monostearate | 5 |
| white vaseline | 94 |
| Total | 100 |

| Prescription example 5 (suppository) | |
|---|---|
| Component | (g) |
| substance of this invention | 0.1 |
| witepsol H-15 | 1.9 |
| Total | 2.0 |

## Claims

1. A pharmaceutical composition containing a water-soluble silicate polymer having a molecular weight of 4,800-2,000,000 and a degree of polymerization of 75-33,000 as an active ingredient and a pharmaceutically acceptable carrier.

2. A pharmaceutical composition according to Claim 1, wherein the water-soluble silicate polymer is a polymerization product of water-soluble silicates.

3. A pharmaceutical composition according to Claim 1 or Claim 2, wherein the water-soluble silicates are alkali metal salts of silicic acid.

4. A pharmaceutical composition according to Claim 3, wherein the silicates are sodium silicates.

5. A pharmaceutical composition according to Claim 1, wherein the water-soluble silicate polymer is a polymerization product of water glass.

6. A process for manufacturing a water-soluble silicate polymer having a molecular weight of 4,800-2,000,000 and a degree of polymerization of 75-33,000, comprising the steps of dissolving a silicate in an aqueous solvent, adding a saccharide to the silicate solution, adjusting the pH of the solution to 4-10, and then drying the solution.

7. A process according to Claim 6, wherein the silicate polymer aqueous solution is dried to a powder.

8. A process according to Claim 7, wherein the solution is dried by lyophilization.

9. The use of a water-soluble silicate polymer having a molecular weight of 4,800-2,000,000 and a degree of polymerization of 75-33,000 for the manufacture of a medicament effective in regulating the function of a living body.

10. The use according to Claim 9, wherein the medicament acts as an anti-allergy agent, an anti-inflammatory agent, an agent for improving peripheral blood circulation, an agent for improving paresthesia or an analgesic agent.

## Patentansprüche

1. Pharmazeutische Zusammensetzung, die ein wasserlösliches Silikatpolymer, das ein Molekulargewicht von 4.800 bis 2.000.000 und einen Polymerisationsgrad von 75 bis 33.000 hat, als aktives Ingrediens und einen pharmazeutisch akzeptablen Träger enthält.

2. Pharmazeutische Zusammensetzung nach Anspruch 1, in der das wasserlösliche Silikatpolymer ein Polymerisationsprodukt wasserlöslicher Silikate ist.

3. Pharmazeutische Zusammensetzung nach Anspruch 1 oder Anspruch 2, in der die wasserlöslichen Silikate Alkalimetallsalze von Kieselsäure sind.

4. Pharmazeutische Zusammensetzung nach Anspruch 3, in der die Silikate Natriumsilikate sind.

5. Pharmazeutische Zusammensetzung nach Anspruch 1, in der das wasserlösliche Silikatpolymer ein Polymerisationsprodukt von Wasserglas ist.

6. Verfahren zur Herstellung eines wasserlöslichen Silikatpolymers, das ein Molekulargewicht von 4.800 bis 2.000.000 und einen Polymerisationsgrad von 75 bis 33.000 hat, umfassend die Schritte Auflösen eines Silikats in einem wäßrigen Lösungsmittel, Zusetzen eines Kohlenhydrats zu der Silikatlösung, Einstellen des pH der Lösung auf 4 bis 10 und dann Trocknen der Lösung.

7. Verfahren nach Anspruch 6, wobei die wäßrige Silikatpolymer-Lösung zu einen Pulver getrocknet wird.

8. Verfahren nach Anspruch 7, wobei die Lösung durch Gefriertrocknung getrocknet wird.

9. Verwendung eines wasserlöslichen Silikatpolymers, das ein Molekulargewicht von 4.800 bis 2.000.000 und einen Polymerisationsgrad von 75 bis 33.000 hat, zur Herstellung eines Medikaments, das zur Regulierung der Funktion eines lebenden Körpers wirksam ist.

10. Verwendung nach Anspruch 9, wobei das Medikament als Mittel gegen Allergien, Mittel gegen Entzündungen, Mittel zur Verbesserung der peripheren Blutzirkulation, Mittel zur Verbesserung von Parästhesie oder als Analgetikum wirkt.

## Revendications

1. Composition pharmaceutique contenant un polymère de silicate soluble dans l'eau ayant une masse moléculaire de 4 800 à 2 000 000 et un degré de polymérisation de 75 à 33 000, servant d'ingrédient actif, et un support acceptable en pharmacie.

2. Composition pharmaceutique selon la revendication 1, dans laquelle le polymère de silicate soluble dans l'eau est un produit de polymérisation de silicates solubles dans l'eau.

3. Composition pharmaceutique selon la revendication 1 ou 2, dans laquelle les silicates solubles dans l'eau sont des sels de métal alcalin et d'acide silicique.

4. Composition pharmaceutique selon la revendication 3, dans laquelle les silicates sont des silicates de sodium.

5. Composition pharmaceutique selon la revendication 1, dans laquelle le polymère de silicate soluble dans l'eau est un produit de polymérisation de verre soluble.

6. Procédé pour fabriquer un polymère de silicate soluble dans l'eau ayant une masse moléculaire de 4 800 à 2 000 000 et un degré de polymérisation de 75 à 33 000, comprenant les étapes de dissolution d'un silicate dans un solvant aqueux, d'addition d'un saccharide à la solution de silicate, d'ajustement du pH de la solution à 4-10, et ensuite de séchage de la solution.

7. Procédé selon la revendication 6, dans lequel la solution aqueuse de polymère de silicate est séchée en une poudre.

8. Procédé selon la revendication 7, dans lequel la solution est séchée par lyophilisation.

9. Utilisation d'un polymère de silicate soluble dans l'eau ayant une masse moléculaire de 4 800 à 2 000 000 et un degré de polymérisation de 75 à 33 000, pour la fabrication d'un médicament efficace dans la régulation de la fonction d'un corps vivant.

10. Utilisation selon la revendication 9, dans laquelle le médicament agit en tant qu'agent anti-allergique, agent anti-inflammatoire, agent pour améliorer la circulation sanguine périphérique, agent pour améliorer la paresthésie ou agent analgésique.
